# EUROPÄISCHE PATENTSCHRIFT

(11) **EP 2 061 432 B1**
(45) Veröffentlichungstag und Bekanntmachung des Hinweises auf die Patenterteilung: **19.12.2012**
(21) Anmeldenummer: 07724734.4
(22) Anmeldetag: 30.04.2007
(51) Int. Cl.: A61K 9/10, A61K 9/107, A61K 31/00, A61K 31/045, A61K 31/125, A61K 31/165, A61K 31/167, A61K 31/192, A61K 31/245, A61K 31/4439, A61K 31/465, A61K 31/60, A61K 47/10, A61K 47/12, A61K 47/14

(54) **NANO-VEHIKEL FÜR DEN TRANSKUTANEN TRANSPORT, DIESE ENTHALTENDE ZUBEREITUNGEN UND DEREN ANWENDUNG**
NANO-VEHICLE FOR THE TRANSCUTANEOUS TRANSPORT, PREPARATION CONTAINING THE SAME AND ITS USE
NANO-VÉHICULES POUR LE TRANSPORT TRANSCUTANÉ, PRÉPARATIONS LES CONTENANT ET LEUR UTILISATION

(30) Priorität: 12.09.2006 DE 102006042742
(43) Veröffentlichungstag der Anmeldung: 27.05.2009
(73) Patentinhaber: Barnikol-Keuten, Doris, 55128 Mainz (DE)
(72) Erfinder: Barnikol-Keuten, Doris, 55128 Mainz (DE)
(74) Vertreter: Müller, Claudia
(86) Internationale Anmeldenummer: PCT/EP2007/003807
(87) Internationale Veröffentlichungsnummer: WO 2008/031465

(56) Entgegenhaltungen:
- WO-A-03/024436
- WO-A-03/045349
- WO-A-2007/022924
- US-A1- 2004 063 794
- US-A1- 2006 057 168

## Beschreibung

### Gegenstand der Erfindung

Die vorliegende Erfindung betrifft Nano- Vehikel zum verbesserten transkutanen Transport, insbesondere von pharmakologisch aktiven Wirkstoffen. Die Vehikel (mit Gefäß-erweitemder bzw. beruhigender Wirkung) umfassen ein oder mehrere Mittel mit an sich hautreizender Wirkung bzw. und lokalanästhetischer Wirkung. Damit können überraschender Weise Wirkstoffe, insbesondere in Form von Mikro-Emulsionen, verbessert lokal / topisch transportiert werden, obgleich eine derartige Kombination das Gegenteil hätte vermuten lassen. Eine verbesserte transkutane Anwendung kann vor allem zu einer lokal sonst nicht zu erreichenden Wirkstoffkonzentration sowie zu einer verkürzten Anwendungszeit und damit langfristig zu einer erheblichen Reduzierung von Nebenwirkungen führen. Je nach aktivem Wirkstoff können somit pharmakologische, insbesondere dermatologische Zubereitungen z. B. bei entzündlichen/ schmerzhaften Zuständen von Haut / Unterhautgewebe bei Säugern hergestellt und angewendet werden.

### Stand der Technik

Es gibt eine Reihe medizinisch hochwirksamer Substanzen, die jedoch nach systemischer Applikation starke nicht akzeptierbare Nebenwirkungen entfalten. Zwei Gruppen von Substanzen sind hierbei besonders interessant, nämlich Lokal-Anästhetika und Endzündungshemmer, speziell nichtsteroidale. Lokal-Anästhetika, systemisch verabreicht, führen bei zu hohem Blutspiegel zu kardialen und zentralnervösen Störungen. Erstere äußern sich in Bradykardie, artrioventrikulärem Block und schließlich Herzstillstand, letztere als Unruhe, Erbrechen, Angstzuständen bis hin zu klonischen Krämpfen (E. Mutschler, G. Geisslinger, H. K. Kroemer, M. Schäfer-Korting "Arzneimittelwirkungen", Wiss. Verlagsges., 2001, ISBN 3-8047-1763-2).

Steroidale Entzündungshemmer können systemisch auf Dauer deshalb nicht gegeben werden, weil sie u. a. eine Osteoporose verstärken, eine Infektionsgefahr erhöhen, gastro-intestinale Blutungen begünstigen, die Wundheilung verzögern, zur Muskel-Atrophie führen, diabetogen wirken und schließlich Schlafstörungen hervorrufen (vergl. Mutschler et al., s. oben).

Systemische Anwendung nichtsteroidaler Entzündungshemmer -auch eine kurzfristige-kann gastro-intestinale Störungen (Erosionen) hervorrufen, bis zu Ulzerationen, Blutungen, Perforationen, Nierenfunktions-Störungen, Blutungsstörungen (Hemmung der Thrombozyten-Aggregation), Schwindel sowie Kopfschmerzen. Die gleichen schwerwiegenden Nebenwirkungen treten nach systemischer Gabe des sehr effektiven Schmerzmittels Acetyl-Salicylsäure auf (vergl. Mutschler et al., siehe oben).

Auf der anderen Seite gibt es lokale entzündliche Störungen, die mit starken Schmerzen und auch Jucken einhergehen. Hierbei sind 2 Fälle zu unterscheiden: Erstens solche Störungen der Haut und zweitens Störungen im unterliegenden bindegewebigen Bewegungsapparat. Zum ersten Fall gehören die peripheren Neuropathien, seien sie bspw. infektiös (Herpes zoster) oder diabetisch bedingt. Dazu zählen aber auch die Dermatiden, insbesondere die Neurodermitis. Zum zweiten Fall gehören bspw. die Arthritiden und Tendino-Pathien, wobei die entzündlichen Veränderungen unterschiedlicher Genese sein können, bspw. rheumatisch oder durch mechanische Einwirkungen. Endergebnis sind stets degenerative Gewebe-Veränderungen.

Die erläuterten lokalen Störungen ließen sich effektiv bekämpfen, wenn es gelänge, die wirksamen Substanzen dementsprechend mit ausreichend hohem Wirkspiegel in die betroffenen Gewebe, sprich in die Haut oder in das betreffende unterliegende Bindegewebe, direkt an den erforderliche Ort zu bringen. Dem stellen sich prinzipiell zwei Hindernisse in den Weg. Einmal die etwa 20 µm d ünne Homschicht der Haut mit ihrem Aufbau aus Hunderten Bilipid- und WasserSchichten parallel zur Hautoberfläche und zum Anderen das für den konvektiven Einwärts-Transport der Wirksubstanzen unzureichende mikrozirkulatorische System.

Mit Hilfe herkömmlicher Gele lässt sich das geschilderte Problem nicht lösen (vergl. J. Rademacher, D. Jentsch, M. A. Scholl, T. Lustimetz, J. C. Frölich: "Diclofenac concentrations in synovial fluid and plasma after cutaneous application in inflammatory and degenerative joint", Br. J. Clin. Pharmacol. 31 (1999) 537-541). Schon besser lassen sich interessierende Wirksubstanzen mit Hilfe von Mikro-Emulsionen durch die etwa 20 µm dünne Homschicht-Barriere bringen. Dies kann an exzidierter menschlicher Haut nachgewiesen werden (vergl. M. Malmstem: "Microemulsions in Pharmaceuticals" in "Handbook of Microemulsion Science and Technology". P. Kumar, K.L. Mittal(eds.), Marcel Dekker 1999, ISBN 0-8247-1979-4, pages 755-771). Gegenüber einfachen Systemen steigert sich im Allgemeinen die Penetration etwa um einen Faktor 5.

Jedoch ist die Steigerung der Homschicht-Penetration für sich allein nicht hinreichend, um die Wirksubstanzen zentimetertief in die unterliegenden Bindegewebe zu bringen. Die Diffusion ist hierfür ein viel zu langsamer Prozess, erfordertich ist ein konvektiver Transport.

In der US 2006/057168 A1 werden Mikro- Emulsionen und Herstellungsverfahren hierfür beschrieben. Darin können pharmazeutische Wirkstoffe enthalten sein wie z. B. Analgetika, und / oder Anästhetika, Sedativa, Hypnotika oder Angstlösende Mittel, Nährstoffe, entzündungshemmende Wirkstoffe, Antihistaminika, Antidepressiva u.ä. Die Mikro-Emulsion dient als Vehikel für die Wirkstoffe.

Die WO 03/045349 A2 betrifft O/W- Emulsionen oder ggf. daraus erhältliche Mikro-Emulsionsgele, welche eine Juckzeiz stillende Wirkung entfachen. Dazu wird der Emulsion eine Kombination aus Polydocanol und einem ätherischen Öl wie Menthol hinzugefügt.

In der US 2004/0063794 A1 wird ein Lösungs-Vehikel, z. B. für eine Mikro-Emulsion, aus speziellen Substanzen zur topischen Verabreichung beschrieben. Dieses Vehikel ist eine eutektische Mischung aus Campher, Menthol und / oder Thymol, speziell für nicht steroidale entzündungshemmende Verbindungen, deren Löslichkeit in der topischen Formulierung erhöht werden soll.

Gemäß der nachveröffentlichten WO 2007/022924 A2 werden Pharmazeutische Zubereitungen, enthaltend eine Wirksubstanz, mit einem Parfüm- oder Geschmacksstoff wie z. B. ein Phenylsalicylat kombiniert zwecks Herabsetzung des Schmelzpunktes des Wirksubstanz. Die Zubereitung kann auch in Form einer Mikro- Emulsion vorliegen.

### Aufgabe der Erfindung

Der vorliegenden Erfindung liegt die Aufgabe zugrunde, ein System bereitzustellen, mit dem ein effektiver Transport von Wirkstoffen, insbesondere entzündungshemmenden, juckreizstillenden und anderen Wirkstoffen mit systemischer Problematik, extern topisch möglich ist.

### Lösung der Aufgabe

Diese Aufgabe wird erfindungsgemäß gelöst durch Mikroemulsionene enthaltend ein Vehikel- System, mit einem oder mehreren Mitteln mit an sich hautreizender Wirkung (Irritantien) und tokalanästhetischer Wirkung, insbesondere mit einem oder mehreren Lokalanästethika zusammen mit einem oder mehreren an sich hautreizenden Mitteln (Irritantien), Wobei das/die Hant reizende (n) Mittel ansgewählt ist/ sind aus Capsaicinoidenr (Vanillyl-Amide (ungesättigter) aliphatischer C₈-C₁₃-Carbonsäuren. Überraschenderweise wurde gefunden, dass paradoxerweise die Anwendung von Irritantien, die die Haut eigentlich reizen, die Effektivität von Wirksubstanzen im Gewebe entscheidend verbessert, wenn zugleich auch Mittel mit lokalanästethischer Wirkung, insbesondere wenn sie zusammen mit Lokalanästhetika, verabreicht werden. Weiterhin wurde überraschend gefunden, dass der weitere Zusatz polarer und/ oder unpolarer Diffusions-Verstärker zu den Zubereitungen die Wirksamkeit darüber hinaus entscheidend verstärkt. Die Erfindung betrifft Zubereitungen, wie wirkstoffhaltige Zubereitungen, in Form einer Mikro-Emulsion, enthaltend ein Vehikel System aus einem oder mehreren an sich hautreizenden Mitteln (Irritantien) und einem oder mehreren lokalanästhetisch wirkenden Mitteln. Derartige Zubereitungen sind vor allem topisch applizierbar.

Die vorliegende Erfindung betrifft insbesondere derartige Zubereitrungen, vor allem Tensid-haltige Zubereitungen in Form einer Mikro- Emulsion, aus einer wässrigen und einer Lipoidphase, welche dadurch gekennzeichnet ist, dass sie eine oder mehrere indikationsbezogene Wirksubstanz(en) und ein System (Transportvehikel) aus einem oder mehreren an sich hautreizenden Mitteln (Irritantien) und lokalanästhetisch wirkenden Mitteln, insbesondere aus einem oder mehreren hautreizenden Mitteln zusammen mit einem oder mehreren Lokalanästhetika aufweist.

Das oder die Hau ist oder sind reizende(n) Mittel, insbesondere hyperämisierende (gefäßerweiterende) Mittel ausgewählt aus Capsaicinoiden, welche auch lokalanästhetisch, d. h. schmerzlindernd wirken.

Das oder die Lokalanäthetika sind vor allem ausgewählt aus Lidocain, Procain, Bupivacain, Polidocanol, Benzocain, Tetracain, Prilocain, Mepivacain, Etidocain, Levobupivacain, Ropivacain, Articain, Fomocain; oder Mischungen hiervon. Die indikationsbezogenen Wirkstoffe können insbesondere ausgewählt werden aus entzündungshemmenden Mitteln, anti- Gicht-Mitteln, anti-Arthritis-Mitteln, anti-Herpes zoster-Mitteln, anti-Neuralgie-Mitteln, anti-Neuropathie-Mitteln, anti-Thrombophlebitis-Mitteln, antiviralen Mitteln, anti-Akne-Mitteln, Schmerzmifteln, Anti-thrombose- Mitteln und juckreizstillenden Mitteln, weiter aus anti-allergischen, anti-Herpes zoster-Mitteln, anti-Neuralgie-Mitteln, anti-Neuropathie-Mitteln, anti-Thrombophlebitis-Mitteln, antiviralen Mitteln, anti-Akne-Mitteln, Schmerzmitteln, Anti-thrombose- Mitteln und juckreizstillenden Mitteln, weiter aus anti-allergischen, anti-infektiösen, anti-hämorrhagischen, anti-histaminischen, anti-koagulierenden, anti-mykotischen, aquaretischen, ödemprotektiven, anti-oxidativen, antiparasitischen, anti-rheumatischen, anti-septischen, anti-infektiösen, spasmolytischen, keratolytischein, Mitose-hemmenden, Muskel-relaxierenden und vasodilatorischen Mitteln, wie z. B. Colchicin, Aciclovir, Teebaum-Öl, Podophyllotoxin, Melissen-Öl, Glycyrrhetinsäure, Cardiospermum-Urtinktur, Tretinoin, Kamille blau, Thymian-Öl, Zimt-Öl, Birken-Öl, Digitoxin, Heparin, Extraktum hippocastani (Aescin), ätherische Öle von Zypresse, Wacholder, Rosmarin, Niouliöl, Patchouliöl, Tea Treeöl, Geranienöl, Basilikumöl, Ysopöl, Fenchelöl, Manukaöl u. ä. je nach Indikation, Flavonoide, Salicylate, Harnstoff, Retinoide, Dithronal(Cignolin), Mahonia-Urtinktur, Teersubstanzen (Kresole, Phenole), Beinwellextrakt; Vitamine oder geeigneten Mischungen hiervon. Ferner können vorteilhafterweise auch ein oder mehrere Wirkstoffe, ausgewählt aus Salicylamid, Acetylsalicylsäure, Salsalat, Iridometacin, Diclofenac, Fenbufen, Ibuprofen, Ketoprofen, Piroxicam, Harnstoff, Thymol, Chlorhexidin, Erythromycin, Clindamycin, Tetracycline, Glucocorticoide, Hirudin und Derivate, Heparine, Clotrimazol, Isoconazol, Ciclopirox, Roter Weinlaub-Extrakt, Glutathion, Cystein, Coenzym Q10, alpha-Lipon-säure, Carvacrol, Campher, Eugenol, Zimt-Aldehyd, Botulinum-Toxine, Nitroglycerin eingesetzt werden. Auch andere Mittel entsprechend gewünschter Indikationen sind möglich, wie nachfolgend beispielhaft erwähnt.

Die Mikro- Emulsion liegt bevorzugt in Formeiner W/O- Mikro-Emulsion vor. Diese kann vor allem weiterhin Zusatzstoffe, ausgewählt aus Cotensiden und / oder Vesikelbildnem; Diffusionsverstärkern, Feuchthattemitteln, Konservierungsmitteln oder Mischungen hiervon enthalten.

Eine besonders bevorzugte erfindungsgemäße Zubereitung umfasst 12 bis 30 Gew. %einer Öl-(Lipoid-)phase,1 bis 35 % nichtionische Tenside; 0,01 bis 15 Gew.% Transport- Vehikel; 0 bis 37%, vor allem 1 bis 37 Gew.% Zusatzstoffe, ausgewählt aus Cotensiden, Vesikelbildnern, Diffusionsverstärkern, 1 bis 20 Gew.% Wasser oder wässrige Phase sowie 0,01 bis 25 Gew.% eines oder mehrerer indikatonsbezogener Wirkstoffe.

Als Diffusionsverstärker eignen sich vor allem eine oder mehrere Substanzen, ausgewählt aus Terpenoiden wie Limonen, Menthol, Azonen, Ölsäure, Cholesterol, Isopropyl-Myristat, Propylenglycol, Harnstoff, Dimethyl-Acetamid, Dimethyl-Formamid, alkanisiertes Pyrrolidon und Dimethyl-Sulfoxid(DMSO).

Als Cotensid wird vorzugsweise ein aliphatischer Alkohol wie Ethanol, Propanol, Isopropanol, Butanol, Hexanol oder Mischungen hiervon gewählt.

Als Vesikelbildner in erfindungsgemäßen Zubereitungen eignen sich ein oder mehrere Substanzen, ausgewählt aus Lecithin aus Soja, Raps, Baumwollesamen, Eigelb; Phosphatidylcholin aus Soja und Eigelb; Phosphatidylethanolamin; Phosphatidylserin, Phospatidylinosit aus Soja, Raps, Baumwollsamen, hydroxyliertes Lecithin, Gemische aus Phosphatidylcholin und Lecithin, Cholesterin und CholesterinDerivaten.

Als Tenside sind insbesondere nichtionische Tenside mit einem HLB- Wert von 2-8 und Tenside mit einem HLB- Wert von 9-18, vorzugsweise in Kombination enthalten. Besonders bevorzugt sind hier Sorbitane und ethoxylierte Sorbitane.

Die erfindungsgemäße Zubereitung wird äußerlich auf geeignete Weise auf das zu behandelnde Areal wie Haut z. B. tropfenweise oder durch Sprühen aufgetragen und dann einmassiert. Das Auftragen kann mittels eines geeigneten Spenders mit Pumpmechanismus oder aus mit einem geeigneten Treibgas, insbesondere Luft, oder mit Sauerstoff angereichertem Treibgas gefüllten Behältnis erfolgen. Derartige Abgabesysteme sind bekannt. Ferner kann die extern topische Anwendung der Zubereitung manuell durch Einreiben erfolgen, wenn sie in gelförmiger oder cremig- festerer Form vorliegt. Eine gelförmige bis fest-cremige Konsistenz ergibt sich insbesondere, wenn die beschriebene topisch applizierbare Zubereitung mit einer Wasserphase gemischt wird, wobei die Zubereitung dann ca. 20 bis 70 oder bis 60 Gew.%, insbesondere >20 bis 50 Gew%, vor allem 30 bis 45 Gew.% Wasser oder wässrige Phase umfasst und eine W/O oder eine O/W- Mikroemulsion darstellt. Erfindungsgemäße Zubereitungen eignen sich zur externen medizinischen, insbesondere auch zur dermatologischen, Anwendung bei Menschen oder Tieren, beispielsweise bei entzündlichen Störungen wie Gicht, Arthritis, Rheuma, Akne, Psoriasis, venösen Erkrankungen, Herpes Zoster, Neurodermitis, Schmerzen, Koagulationsstörungen, Tendinopathien, Neuropathien, aktivierten Arthrosen, viralen, bakteriellen, mykotischen Infektionen, Allergien, Muskelverspannungen, komplexes regionales Schmerz- Syndrom (CRPS) u. a. m. Ggf. kann auch ein pflegerischer Effekt, z. B. bei Akne- Haut erfolgen.

Im beschriebenen Vehikel System mit lokalanästhetischem und Hautreizendem, insbesondere hyperämiesierenden, Mittel können insbesondere ein oder mehrere Capsaiciode, ggf. auch mit weiteren Hautirritantien eingesetzt werden. Geeignete anwendungsbezogene Wirkstoffe können z. B. aus Tretinoin, Retinoiden, Zimt-Öl, Thymian-Öl, Harnstoff, Glycyrrhetinsäure, Diclophenac, Aciclovir, Melissenöl, Colchicin, Birkenöl, Teebaum-Öl, Podophyllotoxin, Cardiospermum-Urtinktur, Tretinoin, Kamille blau, Digitoxin, Heparin, Extrakturn hippocastani(Aescin), ätherischen Ölen von Zypresse, Wacholder Rosmarin, Flavonoiden, Salicylaten, Retinoiden, Dithronal(Cignolin), Mahonia-Urtinktur, Teersubstanzen(Kresole, Phenole) oder geeigneten Mischungen hiervon oder anderen dem Fachmann bekannten (z. B. wie beschrieben) und geeigneten Mitteln gewählt werden.

### Nähere Erläuterung der Erfindung

Das erfindungsgemäße Vehikel-System aus hautreizendem/n Mittel/n (Irritantien) und lokalanästhetisch wirkenden Mittel/n (Lokalnästhetika) wird mittels einer Zubereitung in Form einer Emulsion mit einem Lipid/ Wasser/ Tensidsystem, ggf. mit Zusatzstoffen, sowie einem oder mehreren der beabsichtigten Indikation entsprechenden Wirkstoffen bereitet und extern / topisch verabreicht. Insbesondere werden an sich bekannte besonders leistungsfähige Mikro-Emulsionen gewählt. Mikro-Emulsionen sind makroskopisch homogene, optisch isotrope und thermodynamisch stabile Systeme aus einer wässrigen und einer Lipoidphase (Ölphase). Weiterhin enthalten sie Emulgatoren (Tenside). Aus Stabilitätsgründen können sie noch Zusatzstoffe, wie Cotenside (z.B. ein kurzkettiger aliphatischer Alkohol), Diffusionsverstärker u.ä. aufweisen. Damit werden O/W-Mikro- Emulsionen oder W/O-Mikro- Emulsionen erhalten, deren Mizellen sehr kleine Partikel mit einer Größe von 20 bis 500 nm aufweisen können.

Erfindungsgemäße Zubereitungen in Form einer Mikro-Emulsion können neben dem Transportvehikel (z. B. 0,01 bis 15 Gew.%) sowie einem oder mehreren indikationsbezogenen Wirkstoffen 0,5 bis 40 Gew. % einer Ölphase zusammen mit 0,05 bis 45 % eines oder mehrerer nichtionischer Tensids/e (Emulgator), eines oder mehrerer Zusatzstoffe, ausgewählt aus Vesikelbildnern wie Phospholipiden, Lecithinen, Alkoholen, insbesondere kurzkettigen aliphatischen Alkoholen, Diffusionsverstärkem, sowie Wasser oder eine wässrige Phase, z. B. 1-20 Gew% aufweisen.

Besonders bevorzugt werden W/O Mikro- Emulsionen für die erfindungsgemäße Zubereitung gewählt.

Diese umfassen neben dem Transport- Vehikel und einem oder mehreren indikationsbezogenen, insbesondere 0,01 bis 25 Gew.% eines oder mehrerer wasserlöslicher oder fettlöslicher Wirkstoffe oder Mischungen hiervon z. B.:
45 bis 90 Gew. %, insbesondere 50 bis 80 Gew. %, einer Öl-(Lipoid-)phase, welche bevorzugt flüssig ist; aber auch gelig sein kann,
0,1 bis 45, insbesondere 5 bis 40 Gew. % nichtionischer Tenside, bevorzugt eine Mischung aus einem oder mehreren W/O- und einem oder mehreren O/W- Tensiden im Verhältnis 1:4 bis 1:1,2. Als Tenside eignen sich insbesondere eine Mischung aus nichtionischen Emulgatoren mit einem HLB Wert von 2-8 und solchen nichtionischen Tensiden mit einem HLB Wert von 9-18 . Ferner können Zusatzstoffe wie nachfolgend beschrieben (z. B. Vesikelbildner, Feuchthalter, Konservierungsmittel und / oder Cotenside, hierunter insbesondere C₁₋₈- Alkohole oder Mischungen hiervon) vorhanden sein. Ferner weist die Mikro- Emulsion eine ausreichende Menge an Wasser oder wässrigen Lösungen, wie 1-20, oder 1-10 % auf. Durch Verdünnen unter Zusatz von Wasser oder einer wässrigen Phase (geringerer Menge) kann diese Zubereitung in eine W/O- Mikro-Emulsion oder bei einem erhöhten Wassergehalt in eine O/W- Mikro- Emulsion überführt werden und erhalten bleiben (z. B. durch Verdünnen mit 0,1 bis 90% einer wässrigen Phase).

Bevorzugt weist die Mikro- Emulsion das Transportvehikel in einer Menge von insgesamt 0,01 bis 15 Gew. %, insbesondere 0,05 bis 10 Gew.% auf. Dabei kann die Menge an Haut- Irritans 0,001 bis 5 Gew.% und die Menge an Lokalanästhetikum 0,01 bis 10 Gew.% betragen.

Die indikationsbezogenen Wirkstoffe werden bevorzugt ausgewählt wie oben beschrieben.

Leistungsfähige Mikro- Emulsionen des oben genannten und beschriebenen W/O Typs sind solche gemäß der DE 102 26 990 A1. Derartige Mikro- Emulsionen weisen Nano-Mizellen auf, ohne dass bestimmte Vernetzer wie z.B. Dimethicon erforderlich wären. Darüber hinaus können gleichzeitig sowohl wasserlösliche als auch fettlösliche Wirkstoffe inkorporiert werden, ohne dass es zu einer Instabilität käme. Die Leistungsfähigkeit derartiger Systeme kann durch einen oder mehrere an sich bekannte unpolare oder polare-Diffusionsverstärker (als Teil einer wässrigen Phase oder einer lipoiden Phase) weiter gesteigert werden. Dabei erhält man je nach Phasendifferenzierung leistungsfähige W/O oder O/W- Emulsionen. Besonders bevorzugt werden insofern Systeme eingesetzt, welche umfassen:
12 bis 30 Gew. %, insbesondere 15 bis 25 Gew. %, einer Öl-(Lipoid-)phase, welche bevorzugt flüssig ist;
1 bis 35, insbesondere 5 bis 25 Gew. % nichtionischer Tenside wie beschrieben, insbesondere O/W- und W/O- Gemische, wie beschrieben;
0,01 bis 15 Gew.%. (Gesamtmenge) Transport- Vehikel- System;
sowie Zusatzstoffe in gewünschter Menge, insbesondere 1 bis 37 Gew.% Zusatzstoffe, ausgewählt aus Cotensiden wie die genannten Alkohole, insbesondere 0,5 bis 5 Gew.%, Vesikelbildnern (0,1 bis 4 Gew.%), Diffusionsverstärkem (1 bis 28 Gew.%) sowie 0,01 bis 25 Gew.% eines oder mehrerer indikatonsbezogener Wirkstoffe, 1 bis 20 Gew. % Wasser oder wässrige Phase. Die Menge an Zusatzstoffen oder weiteren Zusatzstoffen (wie Feuchthalter, Konservierungsmittel) richtet sich hier je nach Art der Zubereitung.

Durch Verdünnen mit Wasser oder einer wässrigen Phase z. B. durch Mischen mit 0,1 bis 70 Gew. % einer Wasserphase kann die oben beschriebene erfindungsgemäße Zubereitung dann ca. 20 oder >20 bis 50 Gew. % wässrige Phase aufweisen und eine W/O oder eine O/W- Mikroemulsion darstellen. Bevorzugt weist die Mikro-Emulsion nach Verdünnen mit Wasser 20-70 Gew.%, vor allem > 20% bis 70, vor allem bis 60 Gew.%. Wasser oder wässrige Phase auf. Je nach Wassermenge liegt die Zubereitung flüssig (z. B. 1-20 Gew.%), oder gelförmig bis cremig-fest (>20 bis 70, vorzugsweise bis 50 Gew.%) vor.

Bei Zusatz von Wasser zu den oben beschriebenen öligen Zubereitungen bilden sich anisotrope Mesophasen in Form von Flüssigkeitsstrukturen, beispielsweise Tubuli oder / und Bilayerschichten in Form von Lamellen. Diese Strukturen beeinflussen die Konsistenz der zunächst flüssigen Zubereitungen: sie werden zunächst gelig und nach weiterer Wasserzugabe schließlich fest. Es entstehen sog. Struktur-Gele und dann Struktur - Cremes. So kann je nach gewünschter Applikationsart eine Zubereitung mit entsprechender Konsistenz bereitgestellt werden: bei vorgesehenem Versprühen verbleibt die Zubereitung flüssig, bei manuellem Auftragen auf die Haut kann je nach Bedarf durch eine entsprechende Wassermenge ein Gel oder eine Creme zur Anwendung kommen.

Nachfolgend werden die einzelnen Komponenten des erfindungsgemäßen Systems, insbesondere in Form einer W/O Mikro-Emulsion, näher beschrieben.

Alle Mengenangaben beziehen sich lauf das Gewicht der Zubereitung, soweit nicht anders angegeben.

### Nähere Beschreibung der Inhaltsstoffe

### 1) Emulsionsbestandteile

Die Lipoidphase kann gewählt werden aus dem Fachmann hierfür bekannten Bestandteilen. Insbesondere umfasst die Lipoid- (Öl-)phase flüssige Öle , wie z.B. die Ester aus Alkancarbonsäuren wie insbesondere Isopropylmyristat, Isopropylpalmitat, Isopropyloleat, Isooctylsterarat, Isononylstearat, n-Butylstearat, n-Hexyllaurat, n-Decyloleat, Isooctylstearat, Isononylstearat, Isononylisonanoat, 2-Ethylhexylpalmitat, 2-Ethylhexyl-Laurat, 2-Hexyldecylstearat, 2-Octyldodecylpalmitat, Ethyloleat, Oleyloleat, Oleylecurat, Erucyloleat sowie synthetische, halbsynthetische und natürliche Gemische solcher Ester oder Mischungen hiervon. Ferner bevorzugt sind Dialkylether, C₆-C₁₈- Fett-Alkohole wie. Lauryl-, Palmitin-, Myristin-, Arachidon, Linolen- und Linolalkohol. Ferner geeignet sind (Tri)glycerinester gesättigter und/oder ungesättigter Alkancarbonsäuren. Hierunter sind besonders synthetische, halbsynthetische und natürliche Öle, z. B. Olivenöl, Mandelöl, Avocadoöl, Sonnenblumenöl, Sojaöl, Erdnussöl, Rapsöl und ähnliche. Auch beliebige Abmischungen einzelner oben genannten Lipoide / Öle und Esteröle sind möglich.

Besonders bevorzugte Öle sind neben den bereits erwähnten auch Isopropylmyristat, Isopropylpalmitat, Isopropylstearat, Isopropyloleat, Olivenöl, Mandelöl, Avo cadoöl, Sonnenblumenöl, Sojaöl, Erdnussöl, Rapsöl, Capryl-Caprinsäure-Triglycerid, Dicaprylylether, Squalan oder Mischungen hiervon.

Als Tenside werden bevorzugt nichtionische Substanzen ausgewählt. Hierzu gehören z. B. ONV Tenside wie ethoxylierte C₈-C₁₈- Fettalkohole wie z. B. Polysorbat 80. Sie weisen insbesondere einen HLB Wert von ca. 9-18 auf. Als W/O Tenside eignen sich solche mit einem HLB- Wert von 2-8 wie Sorbitan- Oleat . Bevorzugt werden Mischungen aus O/W und W/O Tensiden eingesetzt, insbesondere im Verhältnis 1:4 bis 1:1,2, bevorzugt 1:3 bis 1:1,2 und insbesondere 1:2 bis 1:1,3. Besonders bevorzugte W/O- und O/W- Tenside sind nicht ionisch und weisen einen HLB- Wert von 3 bis 7 bzw. 9 bis 18 auf. Bevorzugt sind sie ausgewählt aus Sorbitan-Ethern /Estern, Ethoxylierten Sorbitan Derivaten. Ferner können die Tenside vorteilhaft gewählt werden aus ethoxylierten C₈-C₁₈- Fettalkoholen, Glyceryl-Ether / Ester von gesättigten und ungesättigten Fettsäuren, ethoxylierten Glyceryl-Estern, bevorzugt Di- und Triglyceride, ethoxylierten Alkylethern, oder Fettalkohol-(C16-C18)-Glukosiden oder geeigneten Mischungen der jeweiligen Tenside. Beispielhaft können hier genannt werden: Sorbitan Derivate wie Sorbitanmonolaurat, Sorbitanoleat und Sorbitantrioleat; ethoxylierte Sorbitan- Derivate, wie vor allem Polyethylenglycol(20)-Sorbitanmonolaurat, Polyethylenglycol(20)sorbitan-Monostearat, Polyethylenglykol(20)-Sorbitanmonooleat; Mono-, di-, tri- Glyceryl - und Polyglyceryl- Derivate, Polyglyceryl Diisostearate, Polyglyceryl-2-Oleylether, Polyglyceryl-6-distarat, Polyglyceryl-4-oleylether; ethoxylierte Glyceryl Ester wie ethoxylierte Triglyceride, z. B. Polyethylenglycol(20)- Glyceryltristearat, ethoxylierte Alkylether, insbesondere Polyethylenglykoldodecylether, Polyethylenglykol-Hexadecylether; Fettalkohol- (C16-C18)-Glukoside wie Sucrose-Stearat, Sucrose-Palmitat, Plantacare, 1200 UP und Plantacare 2000 UP, ethoxylierte Fettalkohole mit 8-18 Köhtenstoffatornen in geraden Ketten, insbesondere (Polyethylenglycol(2) -Stearylether (Steareth-2), Stearech13 bis Steareth-20, von Oleth-3 bis Oleth-15., von Cetech13 bis Cetech-20, von Ceteareth 12 bis Ceteareth-30.

Die Menge an nichtionischen Tensiden (eine oder mehrere Verbindungen) in den Zubereitungen beträgt 0,1 bis 45 Gew. % , bevorzugt 1- 45 Gew. %, insbesondere 1- 40 Gew. %, vor allem 1 bis 35 Gew.%, oder auch 5 bis 35 Gew. %, bezogen auf das Gesamtgewicht der Zubereitung.

Bevorzugt sind Cotenside enthalten. Diese werden ausgewählt aus Alkoholen, wie C₁₋₈- Alkoholen wie Ethanol, Propanol, Isopropanol, Butanol oder aus zweiwertigen Alkoholen wie Glykole z.B. Propylenglykol, 1,2-Oktandiol, 1,2- Hexandiol. Die Menge an Alkohol(en) in den Zubereitungen beträgt 0,1 bis 15 Gew. %, vorzugsweise 1 bis 15 Gew. %, besonders bevorzugt 5- 15 Gew. %, insbesondere 5 bis 10 Gew. % bezogen auf das Gesamtgewicht der Zubereitung.

Es ist auch vorteilhaft, wenn zusätzlich 0 bis 30 Gew. %, bevorzugt 0,01 bis 20 Gew. %, Zusatzstoffe vorhanden sind, die insbesondere ausgewählt sind aus Diffusionsverstärkem, Feuchthaltern und Konservierungsmitteln. Weiterhin können als Zusatzstoffe Vesikelbildner wie Lecithine, Phospholipide, Phosphatidylcholine oder Derivate oder Mischungen hiervon, wie Lecithin aus Pflanzen (Soja, Raps, Baumwollsamen) und Eigelb; Phosphatidylcholin aus Soja und Eigelb; Gemische aus Phosphatidylcholin und Lecithin in unterschiedlichen Verhältnissen wie NAT-Produkte, Phosphatidylethanolamin; Phosphatidylserin; Phospatidylinosit aus Soja, Raps, Baumwollsamen; hydroxyliertes Lecithin vorhanden sein. Die Menge an Vesikelbildnern beträgt bevorzugt 0,01 bis 10, bevorzugt bis 8 Gew. %, vor allem 0,1 bis 8 Gew. %, bevorzugt 0,5- 5 Gew. %, insbesondere 1- 5 Gew. % bezogen auf das Gesamtgewicht der Zusammensetzung.

Weiterhin können Konservierungsmittel oder Mischungen vorgenannter Zusatzstoffe enthalten sein.

Besonders bevorzugt sind hier Diffusionsverstärker, Cotenside, Vesikelbildner oder Mischungen hiervon enthalten.

In einer besonders bevorzugten Ausführungsform werden lipophile unpolare und/oder polare, besonders schwach polare Diffusionsverstärker eingesetzt. Diese finden sich bspw. in der Gruppe der Terpenoide. Ein effektiver unpolarer Diffusions-Verstärker ist bspw. Limonen; lipophile schwach polare Diffusions-Verstärker sind bspw. Menthol, Azone, Ölsäure, Cholesterol und Isopropyl-Myristat. Hydrophile stark polare Diffusions-Verstärker sind bspw. Propylenglycol, Harnstoff, Dimethyl-Acetamid, Dimethyl-Formamid, alkanisiertes Pyrrolidon und Dimethyl-Sulfoxid (DMSO).

Diffusions-Verstärker können mit einem Gehalt zwischen 0,01 und 50,0 Gew.%, besonders zwischen 3,0 und 7,0 Gew.% und insbesondere von 4,0 bis 6,0 Gew.% eingesetzt werden.

Eine besondere bevorzugte Ausführungsform erfindungsgemäßer Zubereitungen umfasst 10-30 Gew.% Lipoid- (Öl-)phase, 3-35 Gew.% nichtionische Tenside, insbesondere eine Mischung aus W/O- und O/W-Tensiden der angegebenen Art, vor allem im angegebenen Verhältnisbereich, 0,5 bis 5 Gew.% Cotenside, insbesondere aliphatische einwertige C₁-C₈-Alkohole, 0,01 bis 5 Gew.% Vesikelbildner, 0,01 bis 10 Gew.% Transportvehikel, 0,01 bis 6 Gew.% indikationsbezogener Wirkstoff ,1 bis 10 Gew.% Wasser oder wässrige Phase, 3 bis 25 Gew.% Diffusionsverstärker.

### 2)Transportvehikel (Vehikel-System)

Zum Zweck des effektiven Weiter-Transports der indikationsbezogenen Wirksubstanzen in die Tiefe werden die Mikro-Emulsionen wie erläutert mit einem System aus hautreizendem Mittel/n (Hautirritans) und lokalanästhetisch wirkenden Mittel/n versehen, das aufgrund seiner Inhaltsstoffe zugleich Gefäß-erweiternd und beruhigend und/ oder schmerzstillend wirkt. Insofern kann auch ein therapeutischer Effekt mittels des Transportvehikels erzielt werden. Demnach wirken die an sich hautreizenden Mittel (auch) insbesondere hyperämiesierend (gefäßerweiternd). Capsaicinoide zählen bevorzugt zur Gruppe der an sich hautreizenden Mittel (HautIrritantien), wobei die Gruppe aus Vanillyl-Amiden (ungesättigter) aliphatischer C₈-C₁₃-Carbonsäuren umfasst ist. Die Gruppe der Capsaicinoide besteht aus den Vanillyl-Amiden (ungesättigter) aliphatischer C₈-C₁₃-Carbonsäuren.

Capsaicin und dessen Derivate sind stark Gefäßerweitemd, indem sie eine Histamin-Freisetzung aus den Mastzellen bewirken. Dies führt zu einer Steigerung der Durchblutung. Darüber hinaus entspeichem Capsaicinoide die peripheren Nerven-Endigungen u. a. vom Transmitter Substanz P; sie lösen dadurch initial eine sehr starke Schmerzreaktion aus und deaktivieren so die Schmerzrezeptoren vollständig. Im übrigen ist Capsaicin völlig untoxisch. Der Einsatz der Capsaicinoide ist deshalb von besonderem Vorteil und Interesse, weil dieses hautreizende Mittel (Irritans) auch selbst zugleich als leicht lokalanästhetisch, nämlich schmerzstillend wirkt und ggf. alleine als Nano- Vehikel zum Einsatz kommen kann. In Kombination mit einem Lokalanästhetikum wird darüber hinaus eine besonders ausgeprägte Effektivität gemäß vorliegender Erfindung erzielt.

Im allgemeinen werden hautreizende Mittel in einer Menge von 0,0001 bis 5 Gew.%, eingesetzt, bevorzugt gewählte Capsaicinoide können mit einem Gehalt zwischen 0,0001 und 5,0 Gew.% eingesetzt, insbesondere zwischen 0,005 und 2,5 Gew.%, ganz besonders zwischen 0,01 und 1,0 Gew.% zur Anwendung kommen. Die Menge an Nicotinaten beträgt z. B,. 0,01 bis 10,0 Gew.%; bevorzugt 0,1 bis 8,0 Gew.%; und insbesondere 0,5 bis 4,0 Gew.%.

Die initiale Reaktion durch das Irritans kann durch gleichzeitige Gabe von Lokal-Anästhetika coupiert werden. Deren Wirkung beruht auf einem anderen Mechanismus: Es werden die Natrium-Kanäle der erregbaren Membranen blockiert und somit jede Erregung unterbunden. Lokal-Anästhetika sind nach ihrer Plasma-Bindung, sprich Wirkungsdauer, nach ihrer Toxizität und nach ihrer Lipophilität auszuwählen. Vertreter dieser Gruppe von Substanzen sind vor allem Procain, Lidocain und Bupivacain, aber auch Polidocanol oder andere wie oben erläutert. Lokal-Anästhetika werden mit einem Gehalt zwischen 0,01 und 10,0 Gew.% angewendet, besonders zwischen 0,1 und 6,0 Gew.%, insbesondere zwischen 0,5 und 8,0 Gew.%.

### 3) Indikationsbezogene Wirkstoffe

Aufgrund der erfindungsgemäß verbesserten Verabreichungsform können insbesondere auch solche Substanzen hier eingesetzt werden, deren systemische Anwendung wegen ihrer starken Nebenwirkungen eingeschränkt ist. Dazu gehört bspw. Colchicin aus der Gruppe der Mitose-Hemmer, das der Bekämpfung der Gicht dient. Dazu gehören auch das Nukleotid-Analog Aciclovir, Teebaum-Öl, Podophyllotoxin (z. B. in Thuja-Öl) und Melissen-Öl, mit welchen der Herpes zoster erfolgreich bekämpft werden kann. Weiterhin lässt sich mit Hilfe des Phytohormons Glycyrrhetinsäure sowie mit Cardiospermum-Urtinktur die Neurodermitis mit Erfolg behandeln. Die Akne kann mit Hilfe von Tretinoin und mit ätherischen Ölen, wie Kamille blau, Thymian-Öl und Zimt-Öl, bekämpft werden. Weitere effektive Schmerzmittel, welche mit dem Vehikel lokal hervorragend eingesetzt werden können, sind Salicylate, wobei sich Birken-Öl als Vorläufer-Substanz besonders eignet. Weitere Wirkstoffe können sein: Digitoxin, Heparin, Extraktum hippocastani(Aescin), ätherische Öle, wie von Zypresse, von Wacholder und von Rosmarin, sowie Flavonoide(z. B. Rutin) lassen sich erfolgreich gegen Thrombo-Phlebitis einsetzen. Harnstoff, Salicylate, Retinoide, Dithronal (Cignolin), Mahonia-Urtinktur und Teersubstanzen (Kresole, Phenole) können mit Erfolg gegen Psoriasis eingesetzt werden.

Weitere indikationsbezogene Wirkstoffe sind z. B. Diflunisal, Salicylamid, Ethenzamid, Acetylsalicylsäure, Salsalat, Iridometacin, Acemetacin, Proglumetacin, Diclofenac, Tolmetin, Lonazolac, Fenbufen, Nabumeton, Ibuprofen, Ketoprofen, Flurbiprofen, Tiaprofensäure, Fenoprofen, Naproxen, Dexketoprofen, Piroxicam, Teno xicam, Metoxicam, Meloxicam, Lornexicam, Mefenaminsäure, Flufenaminsäure, Nifluminsäure, Nabumeton, Azapropazon, Aceclofenac, Azapropazon, Oxyphenbutazon, Phenylbutazon, Mofebutazon, Paracetamol, Nifluminsäure, Bufexamac, Propylphenazon, Metamizol, Harnstoff. Thymol, Chlorhexidin, Fusidinsäure, Mupirocin, Sulfadiacin, Erythromycin, Clindamycin, Tetracycline, Medocyclin, Tyrothricin, Gentamycin, Neomycin, Bacitracin, Chloramphenicol, Polymyxin, Kanamycin, Glucocorticoide, Hirudin und Derivate, Heparine, insbesondere niedermolekulare, Clotrimazol, Bifonazol, Econazol, Fenticonazol, Isoconazol, Oxiconazol, Sertaconazol, Tioconazol, Miconazol, Ketoconazol, Itraconazol, Fluconazol, Voriconazol, Sertaconazol, Terbinafin, Naftifin, Amorolfin, Amphotericin B, Griseofulvin, Flucytosin, Ciclopirox, Nystatin, Natamycin Thiocartionate, Roter Weinlaub-Extrakt, Glutathion, Cystein, Coenzym Q10, alpha-Liponsäure, Bufexamac, Glycyrrhetinsäure, Thymol, Carvacrol, Campher, Eugenol, Zimt-Aldehyd, Salicin, Valciclovir, Penciclovir, Famciclovir, Idoxaridin, Bivudin, Trifluridin, Vidarabin, Tromantadin, Foscarnet, beta-Interferon, Tubocurarin, Alcuronium-Chlorid, Pancuronium-Bromid und ähnliliche Substanzen, Suxamethonium-Chlorid, Dentrolen, Botulinum-Toxine, Nitroglycerin oder geeignete Mischungen hiervon.

Die Menge an einzelnen anwendungsbezogenen Wirkstoffen ist dem Fachmann bekannt und kann variiert werden. In der nachfolgenden Tabelle 1 sind einige Beispiele erfindungsgemäß einsetzbarer Wirkstoffe und deren Anwendungsmengen (jeweils nachfolgend allgemeiner Bereich, bevorzugter Bereich und besonders bevorzugter Bereich) angegeben.

**Tabelle 1**

| Indikationsgebiet | Wirkstoff | Anwendungsmenge allgemein; Bevorzugt; besonders bevorzugt |
|---|---|---|
| Gicht | Colchicin | 0,00001 bis 3,0 Gew.%; 0,0001 - 2,0 Gew.%; 0,001- 0,8 Gew.%; |
| Herpes zoster | Aciclovir | 0,0001 bis 10,0 Gew.%; 0,001 bis 5,0 Gew.%; 0,01 bis 2,0 Gew.%. |
| Neurodermittis | Glycyrrhetinsäure | 0,0001 bis 10,0 Gew.%; 0,001 bis 5,0 Gew.%; 0,01 bis 3,0 Gew.%. |
| Akne | Tretinoin und Retinoide (VitaminA,-Säuren, Derivate | 0,0001 bis 5,0 Gew.%; 0,001 bis 3,0 Gew.%; 0,01 bis 0,5 Gew.%;. |
| Schmerz | Salicylate (z. B. Birken-Öl, Hydroxyethyl-Salicylat) | 0,1 bis 25,0 Gew.%; 1,0 bis 20,0 Gew.%; 10,0 bis 20.0 Gew.%. |
| anti-viral | Podophyllotoxin, z. B. in Thuja-Öl; | 0,001 bis 10,0 Gew.%; 0,01 bis 3,0 Gew.%; 0,15 bis 0,5 Gew.%. |
| anti-phlogistisch | Kamille blau | 0,01 bis 25,0 Gew.%; 0,1 bis 20,0 Gew.%; 1,0 bis 15,0 Gew.%. |
| Akne | Zimt-Öl | 0,005 bis 12,5 Gew.%; 0,05 bis 10,0 Gew.%; 0,5 bis 8,0 Gew.%. |
| Akne | Thymian-Öl | 0,005 bis 13,0 Gew.%; 0,04 bis 9,0 Gew.%; 0,4 und 7,5 Gew.%. |
| Neurodermitis | Cardiospermum | 0,1 bis 40,0 Gew.%; 0,5 bis 30,0 Gew.%; 5,0 bis 20,0 Gew.%. |
| Thrombo-Phlebitis | Digitoxin | 0,000001 bis 1,0 Gew.%; 0,001 bis 0,5 Gew.%; 0,001 bis 0,1 Gew.%. |

| Indikationsgebiet | Wirkstoff | Anwendungsmenge allgemein, bevorzugt; besonders bevorzugt; |
|---|---|---|
| Thrombose | Heparin | 2000 bis 250000 I.E.; 5000 bis 20000 I.E.; 6000 bis 15000 I.E. |
| Psoriasis | Mahonia-Urtinktur | 0,1 und 30,0 Gew.%; 1,0 bis 25,0 Gew.%; 5,0 und 15,0 Gew.%. |
| Thrombose | Extraktum hippocastani (mit 3-5 Gew.% Aescin) | 0,1 bis 40,0 Gew.%; 1,0 bis 30,0 Gew.%; 3,0 bis 20,0 Gew.%. |
| Schwellung | Zypressen-Öl | 0,1 und 40,0 Gew.%; 1,0 bis 30,0 Gew.%; 5,0 bis 20,0 Gew.%. |
| Schwellung | Wacholder-Öl | 0,1 bis 40,0 Gew.%; 1,0 bis 30,0 Gew.%; 5,0 bis 20,0 Gew.%. |
| Schwellung | Rosmarin-Öl | 0,1 bis 40,0 Gew.%; 1,0 bis 30,0 Gew.%; 5,0 und 20,0 Gew.%. |
| Schwellung | Flavonoide | 0,01 bis 30,0 Gew.%; 0,1 bis 15,0 Gew.%; 0,5 und 8,0 Gew.%. |
| Psoriasis | Harnstoff | 1,0 bis 30,0 Gew. %; 2,0 bis 20,0 Gew.%; 5,0 bis 10,0 Gew.%. |
| Neuro-Dermitis | Corticosteroide | 0,00001 bis 10,0 Gew.%; 0,0001 bis 5,0 Gew.%; 0,001 bis 4,0 Gew.%. |
| Psoriasis | Dithranol | 0,01 bis 30,0 Gew.%; 0,05 bis 20,0 Gew.%; 0,1 und 10,0 Gew.%. |
| Herpes Zoster | Melissen-Öl | 0.01 bis 25,0 Gew.%; 0,01 bis 17,0 Gew.%; 7,0 bis 13,0 Gew. % |
| Psoriasis | Steinkohlenteer | 0,01 bis 30,0 Gew.%; 0,1 bis 20,0 Gew.%; 2,0 und 15,0 Gew.%. |

Wie insbesondere auch aus den Beispielen ersichtlich, ist aufgrund der lokalen Anwendung insgesamt viel weniger Indikationswirkstoff für eine erfolgreiche Therapie nötig als bei oraler Anwendung.

Besonders bevorzugte Hautreizende Mittel sind ein oder mehrere Capsacinoide, oder auch ein oder mehrere Capsacinoide insbesondere in Kombination mit Lidocain. Besonders bevorzugtes Öl ist Isopropylmyristat (welches auch einen Diffusionsverstärkenden Effekt aufweist). Besonders bevorzugte Diffusionsverstärker sind DMSO und ätherische Öle wie Limonen oder Kombinationen hiervon oder mit den vorgenannten Komponenten. Bevorzugte Tenside sind Mischungen aus ethoxylierten Tensiden wie Polysorbat 80 und nicht ethoxylierten Sorbitanestem wie Oleaten, vor allem mit einem Überschuss an ethoxylierten Mitteln.

### Herstellung erfindungsgemäßer Zubereitungen

Erfindungsgemäße Zubereitungen werden hergestellt, indem man die Ölphase, enthaltend das oder die Tenside, ggf. Vesikelbildner, den oder die Alkohole sofern vorhanden sowie unpolare lipidlösliche Diffusionsverstärker sofern vorhanden und ggf. darin vorhandene fettlösliche Wirkstoffe, sowie Bestandteile des Nano- Vehikel Systems, sofern darin löslich, mit einer Wasserphase, ggf. enthaltend den oder die wasserlöslichen Wirkstoffe / Bestandteile des Nano- Vehikel-Systems sofern darin löslich, bei nicht wesentlich erhöhten Temperaturen, insbesondere von 10 bis 30 C miteinander intensiv vermischt, und die so erhaltene Mikro- Emulsion erhält. Für den Fall eines Ölanteils von 45 und mehr Gew.% (wobei W/O Mikro-Emulsionen erhalten werden), können diese anschließend ggf. mit einer Wasserphase, die ggf. weitere wasserlösliche Wirkstoffe aufweisen kann, in eine sekundäre W/O- oder eine sekundäre O/W- Mikro- Emulsion überführt werden.

### Anwendungen

Je nach indikationsbezogenem Wirkstoff eignen sich erfindungsgemäße Zubereitungen zur Anwendung bei Säugern, insbesondere beim Menschen oder bei Tieren, z. B. Pferden, Hunden als medizinische, insbesondere dermatologische, Zubereitungen zur topischen Behandlung einer allergisch, bakteriell, immunologisch, durch äußere Einflüsse beeinträchtigten, irritierten oder beschädigten und degenerierten Haut, oder von entzündlichen Prozessen in unterliegenden geweblichen Bereichen von Säugern. Zur genauen Dosierung werden die Zubereitungen entweder tropfenweise auf die Haut aufgegeben -dies vor allem in der Human-Medizin auf die Nackthaut - oder aufgesprüht (dies vor allem in der Veterinär-Medizin nach dem Rasieren oder auch sehr sorgfältigen Scheren bei Fellhaut). Eine weitere Möglichkeit besteht darin, die Zubereitungen mit Hilfe eines Pinsels oder einer Bürste gegen die sorgfältig geschorenen Haare in die Fell-Haut einzutupfen. Bevorzugt kann das aufgetragene Material durch angemessenes Massieren eingearbeitet werden. Das Versprühen kann durch Anwendung geeigneter Sprühvorrichtungen mit oder ohne Treibgas (mit Luft oder auch mit Sauerstoff bzw. Sauerstoff angereichertem Treibgas) erfolgen. Die Zubereitung ist insbesondere flüssig, kann aber auch gelförmig bis cremig- fest sein. Sie eignet sich zur Anwendung bei entzündlichen Störungen / Erkrankungen von Haut, Gelenken, wie die Anwendung bei Akne, Neurodermitis, Psoriasis, geweblichen Störungen wie Gicht, Arthrithis, Thrombosen. Zur medizinischen, insbesondere dermatologischen, Anwendung erfolgt der Einsatz der indikationsbezogenen Mittel in dem Fachmann bekannter und jeweils indizierter Menge, je nach Schwere und Erfordernis der Störung.

Die Erfindung wird anhand der nachfolgenden Beispiele 1-3 (Herstellung) und 4-11 (Anwendung) näher erläutert.

### Beispiele

### A. Herstellung: Folgende Zubereitungen wurden, wie beschrieben, hergestellt:

**Tabelle 2**

| Beispiel 1 Inhaltsstoff | 1 | 2 | 3 |
|---|---|---|---|
| Triamcinolon-Acetonid | 0,06 g | --- | --- |
| Diclofenac-Na | 0,4 g | 0,9 g | --- |
| Capsaicin | 0,003 g | 0,006 g | 0,005 g |
| Dimethyl-Sulfoxid | 1,5 g | 0,3 g | 2,0 g |
| Limonen | 0,07 g | 0,25 g | 0,3 g |
| Lecithin | 0,02 g | 0,02 g | 0,02 g |
| Ethanol | 0,2 g | 0,2 g | 0,2 g |
| Aqua dest. | 0,3 g | 1,5 g | 0,11 g |
| Polysorbat 80 | 1,5 g | --- | --- |
| Polysorbat 60 | --- | 1,0 g | --- |
| Polysorbat 40 | --- | --- | 0,87 g |
| Sorbitan-Oleat | --- | --- | 0,22 g |
| Sorbitan-Palmitat | 0, 33 g | --- | --- |
| Sorbitan-Laurat | --- | 0,29 g | --- |
| Lidocain-HCl | --- | 0,24 g | 0,24 g |
| Polidocanol | --- | --- | 0,48 g |
| Bupivacain | 0,12 g | --- | --- |
| Aciclovir | --- | --- | 0,05 g |
| Melissenöl | --- | --- | 0,1 g |
| Isopropylmyristat | Ad 6,0 g | Ad 6,0 g | Ad 6,0 g |

### B. Anwendung

### Beispiel 4: Degeneriertes Handgelenk im akuten Schub

Bei einem 71-jährigen Mann, der aufgrund eines Aortenklappenfehlers und eines Aorten-Aneurysmas marcumarisiert (1/2 Tbl. Pro Tag) ist, traten starke Beschwerden insbesondere im rechten Handgelenk auf (vermutlich aufgrund jahrelangen Kontaktes mit heißem und kaltem Wasser wegen der Tätigkeit in seiner Wäscherei). Eine systemische Anwendung von Diclofenac führte nicht nennenswert zu einer Verbesserung. Als Befund fanden sich starke Kontrakturen beidseits des 4.-5. Fingers mit starker irreversibler Verkrümmung (Flexion), sowie stärkste Schmerzen im rechten Handgelenk.

Die Behandlung erfolgte zunächst mit einer erfindungsgemäßen Zubereitung, enthaltend Diclofenac, Lidocain x HCl, Capsaicin in Dosierungen von 10 mg, 10 mg bzw. 0,02 mg. Mit dieser Dosierung wurden innerhalb von 14 Tagen 3 Behandlungen durchgeführt. Dann wurde die Dosierung in der erfindungsgemäßen Zubereitung auf 20 mg, 10 mg bzw. 0,02 mg gesteigert und 3 Behandlungen innerhalb von einer Woche durchgeführt. Anschließend wurden die Dosierungen weiter auf 50 mg, 10 mg bzw. 0,05 mg verändert und damit 2 Behandlungen durchgeführt. Im Anschluss daran wurden die Dosierungen auf 50 mg, 10 mg bzw. 0,5 mg verändert und damit weitere 2 Behandlungen, unter Zusatz von 20 mg Pfefferminz-Öl durchgeführt. Es folgte anschließend eine letzte Behandlung mit einer Dosis von 75 mg Diclofenac bei gleichbleibender Dosierungen der anderen Substanzen. Während der Behandlungen hat der Patient täglich 1 Tablette mit Weihrauch eingenommen. Nach allen 11 Behandlungen mit steigenden Dosierungen war als Endergebnis der Patient beschwerdefrei; eine Einnahme von Schmerztabletten war nicht mehr erforderlich.

### Beispiel 5: Behandlung eines Fersensporns

Ein 72-jähnger Mann mit starken Schmerzen in der rechten Ferse während des Gehens, welche bis zum Knie heraufzogen, wies einen röntgenologisch verifizierten Fersenspom auf. Zunächst wurde die schmerzhafte Druckstelle mehrere Tage mit Gutta-Plast keratolytisch auf einer Fläche von etwa 3 cm x 3 cm behandelt. Die erweichte Hom-Schicht ließ sich gut bis auf die eigentliche dünne Haut abtragen. Darauf erfolgte die tägliche Behandlung mit einer erfindungsgemäßen Zubereitung enthaltend Diclofenac, Capsaicin, Lidocain. Es wurden über 14 Tage mittels einer Kanüle (Größe 2) insgesamt etwa 10 Tropfen (das entspricht 60 mg) der Zubereitung zu beiden Seiten des Fußes unterhalb der Knöchel sowie auf die schmerzhafte Stelle aufgebracht und durch Massieren mit 2 fingerlingbedeckten Fingern wenige Minuten lang in die Haut eingearbeitet. Somit gelangten pro Behandlung etwa 10 mg Diclofenac auf die Haut. Bereits nach 14 Tagen trat eine erhebliche Schmerzminderung ein. Deshalb wurden die Behandlungen auf 2-3 pro Woche reduziert. Weitere 14 Tage später konnte der Patient wieder unbehindert seiner Arbeit nachgehen und war bei der reduzierten Behandlung nach insgesamt 6 Wochen beschwerdefrei. Unerwünschte Nebenwirkungen traten nicht auf.

### Beispiel 6: Anwendung bei Herpes zoster

Behandelt wurde eine 58-jährige Frau, die einen ausgeprägtem Herpes Zoster im TH4 Dermatom rechts aufwies. Zuvor war sie bereits mit Zostex 7 Tage vorbehandelt worden. Die Schmerzen mussten mit 400 mg Tramadol ret. behandelt werden. Die Haut-Efflorezenzen waren extrem stark ausgeprägt.

Neben der Intensivierung der Schmerztherapie mit 2 x 30 mg Morphin wurde eine Therapie mit einer erfindungsgemäßen Zubereitung (enthaltend Aciclovir, Lidocain, Polidodecanol) begonnen. Es wurde auf das gesamte befallene Dermatom die Zubereitung Rezeptur-Beispiel 3 aufgetragen. Als Treibgas für die Zerstäubung wurde Sauerstoff verwendet. Die Zubereitung wurde breitflächig über dem befallenen Dermatom aufgetragen. Eine Behandlung dauerte ca. 30 Minuten. Es wurden etwa 0,5 ml der Zubereitung mit einem Applikator aufgesprüht. Bei der Patientin wurden 8 Behandlungen über 2 Wochen durchgeführt.

Bereits nach 4 Tagen begann die Haut deutlich zu heilen. Der Schmerz ließ bereits nach 6 Tagen soweit nach, dass das Morphin durch 100 mg Tramadol ersetzt werden konnte. Nach 2 Wochen war die Haut bis auf einige wenige Restkrusten abgeheilt. Nach 3 Wochen war die Patientin schmerzfrei und die Haut war vollkommen abgeheilt.

Beispiel 7: Eine 69-Jährige Frau hatte durch einen Treppensturz eine pertrochantare Schenkelhalsfraktur und eine Humeruskopf-Trümmerfraktur jeweils rechts erlitten. Diese heilte nach zweiwöchiger Ruhigstellung und Osteosynthese ohne wesentliche Fehlstellung konservativ aus, jedoch war die Schulter in der Zwischenzeit weitgehend eingesteift. Es bestanden erhebliche nächtliche Schmerzen.

In einer 6-wöchigen Phase (4 Wochen nach dem Sturz) wurde die Patientin 5-mal einer umfassenden Triggerpunkt-Infiltration der Schulter-Umgebungs-Muskulatur unterzogen, ergänzt durch Infiltration des Acromio-Clavicular-Gelenkes (ACG) und der Schultergelenk-Kapsel mit Carbostesin 0,5 %. Nach jeder Therapiesitzung wurde noch eine vorsichtige Schulter-Mobilisation durch post-isometrische Relaxation der Schultermuskirlatur in mehreren Ebenen durchgeführt. Zusätzlich wurde mehrmals das ACG mobilisiert.

Nach Abschluss dieser Behandlungsphase hatte der Spontanschmerz auf ein erträgliches Maß abgenommen. 2 Monate später bestand noch Bewegungsschmerz in der Schulter. Das ACG war stark druckschmerzhaft. Es wurde sodann mit der Behandlung mit einer erfindungsgemäßen Zubereitung begonnen. Innerhalb von 7 Tagen wurde insgesamt 5-mal das Areal über dem ACG und über dem M. deltiodes für jeweils 20 min. eingesprüht (Lidocain 0,48 g, Capsaicin 0,006 g, Diclofenac 0,9 g in einer erfindungsgemäßen Mikroemulsion ad 6 g). Bereits einen Tag nach der ersten Behandlung war der Druckschmerz über dem ACG geringer geworden. Nach einem weiteren Tag war er verschwunden. Nach Abschluss der Behandlungsserie war auch der Bewegungsschmerz verschwunden. Lediglich bei ausladenden Bewegungen trat endgradig Schmerz auf.

Eine weitere spätere Behandlung der Patienten wegen einer Überwärmung und Kapselschwellung des linken Kniegelenkes erfolgte durch Spray-Behandlung der Knieregion von 20 min Dauer mit der oben genannten Zubereitung. 10 Tage danach war die Kapselschwellung verschwunden, die Hauttemperatur über dem Knie wies keinen Unterschied zur gesunden Seite auf. Die Flexion war normal. Zur Sicherheit wurde eine weitere prophylaktische Therapiesitzung mit der oben genannten Zubereitung durchgeführt.

### Beispiel 8: Behandlung des Komplexen regionalen Schmerz-Syndroms (CRPS)

Eine 29- jährigen Frau, der 8 Jahre zuvor ein Ganglion aus dem dorsalen Handgelenkspatt rechts operativ entfernt worden war, hatte eine Recidiv- OP, wobei gleichzeitig ein Hautast des N. radialis über dem Metacarpale 1 verödet wurde. Die bisherigen Schmerzen waren postoperativ weiter vorhanden und nahmen im Verlauf von zwei Monaten weiter zu. Zusätzlich traten nächtlicher Ruheschmerz auf. Die Beweglichkeit im Handgelenk nahm ab und es kam zu einer Anschwellung über dem ehemaligen Operationsgebiet. Physiotherapie verstärkte die Beschwerden. Die Patientin bekam eine Unterarm-Handgelenks-Manschette zur funktionellen Ruhigstellung des Handgelenks und als analgetische Medikation Ibuprofen, später Diclofenac. Lokal wurde Diclofenac-lontophorese angewendet. Da keine Änderung eintrat, wurde zusätzlich Cortison systematisch für 6 Wochen verordnet. Dies alles blieb ohne jeden therapeutischen Effekt. Inzwischen stellten sich auch vegetative Symptome wie Marmorierung der Haut, vermehrte Handschweißbildung und eine Temperaturdifferenz re. gegen li. ein. Im Knochenszintigramm zeigte sich eine vermehrte Anreicherung im Handwurzelbereich vor allem im radialen Handbereich. Sodann erhielt die Frau insgesamt 12 Behandlungen im dorsalen und radialen Handgelenksbereich von jeweils 15-20 min Dauer mit einer erfindungsgemäßen Rezeptur aus 0,9 g Diclofenac, 0,006 g Capsaicin und 0,8 g Lidocain ad 6 g Mikroemulsion. Nach der Hälfte der Anwendungen waren die Schmerzen in Ruhe verschwunden. Nach Abschluß der Therapie bestanden weder Spontan- noch Bewegungsschmerz.

### Beispiel 9: Akuter Knieschmerz bei Außenbandzerrung nach Sturz

Eine 50jährige Frau zog sich nach einem Sturz eine Außenbandzerrung des linken Kniegelenkes zu. Neben einem mäßig gradigen Ruheschmerz verspürte sie beim Gehen starke und beim Treppengehen sehr starke Schmerzen an der Außenseite des linken Kniegelenkes. Es wurden sodann insgesamt 4 Behandlungen des linken Knies beidseits im lateralen Gelenkbereich großflächig durchgeführt. Die Behanglungsdauer betrug jeweils 30 min mit einer erfindungsgemäßen Rezeptur aus 0,9 g Diclofenac, 0,006 g Capsaicin und 0,9 g Lidocain ad 6 g Mikroemulsion. Während der Behandlungszeit nahm die Patientin keine analgetischen Medikamente ein. Bereits am 1. Tag nach Behandlungsbeginn waren die Schmerzen um 30% reduziert. Am Ende der Behandlung war die Patientin beim Gehen auf ebener Strecke nahezu schmerzfrei.

### Beispiel 10: Verschiedene Behandlungen

In nachfolgender Tabelle 3 ist die Behandlung unterschiedlicher Indikationen von insgesamt 82 Fallbeispielen (Menschen) und deren Ergebnis dargestellt. Die Behandlungen erfolgten mit einer erfindungsgemäßen Zubereitung gemäß Beispiel 7-9 bzw. mit einer erfindungsgemäßen Zubereitung, enthaltend 0,005 g Aciclovir, 0,006 g Capsaicin, 0,24 Lidocain bzgl. der Indikation "Herpes Zoster".

**Tabelle 3**

| Indikation | Fallzahl | erfolgreich | erfolglos |
|---|---|---|---|
| LWS- Syndrom | 9 | 6 | 3 |
| WS- Syndrom | 4 | 3 | 1 |
| Epikondylitis | 11 | 9 | 2 |
| HWS- Syndrom | 12 | 7 | 5 |
| Arthrose oberes Sprunggelenk | 9 | 7 | 2 |
| Arthritis Kniegelenk | 6 | 4 | 2 |
| Arthritis daumengrundgelenk | 9 | 5 | 4 |
| Arthirtis Hüfte | 6 | 5 | 1 |
| Handgelenkschmerzen | 4 | 3 | 1 |
| Herpes Zoster | 12 | 12 | 0 |
| Ergebnis | 82(100) | 61 (74%) | 13 (26%) |

### Beispiel 11:

Im folgenden wurden verschiedene Personen A-S mit den jeweils angegebenen Diagnosen erfindungsgemäß mit Zubereitungen gemäß Beispiel 7-9 wie in Tabelle 4 angegeben behandelt, wobei die dabei erzielten Ergebnisse ebenfalls aus Tabelle 4 entnommen werden können. Hieraus ist ersichtlich, dass mit erfindungsgemäßen Zubereitungen die angegebenen Zustände erfolgreich behandelt werden können.

### Nacken / Hals:

| | |
|---|---|
| A. 93J., w., | chron. Nacken- u. Unterkieferschm. li. |
| B:27J., w. | recidiv. Nacken- u. Gesichtsschmerz |
| C:71J., m. | akuter Nacken- u. Thoraxschmerz |
| D:30J., w. | akuter Nackenschm. bei Kopfgel.-Block. |
| E:38J., w | Akuter Schmerz im Nacken li. u. im ZTÜ (zervikal-thorakaler Übergang) |

### Schulter/Hand:

| | |
|---|---|
| F: 42J., w. | Schutterschmerz nach Sturz |
| G: 69J., w | Schulterschmerz nach Humeruskopf-Fraktur |
| H: 72J., w | .Schultergel.-Arthropathie b. Ochronose |
| I: 36J., w. | Periarthritis humeroscapularis |
| K: 65J., w. | aktivierte Arthrose des Schultergelenks, aktivierte Arthrose, Daumensattelgelenk |

### Ellenbogen/Hand:

| | |
|---|---|
| L: 56J ,m, | Epicondyl-Algie re., Epicondyl-Algie li. u. Insertionstendinopathie der Bicepssehne li. |
| M:71J, m : | Handgelenk-Beschwerden |
| N:29J, w: | CRPS re., Handgelenk nach Ganglion-OP. |

### Oberschenkel:

| | |
|---|---|
| 0:81J, m, | mixed pain syndrome' nach OP, eines Leiomyosarkoms am re. Oberschenkel (Attackenschmerz/ myofascialer Schmerz |

### Knie:

| | |
|---|---|
| P:71 J,w, | aktivierte Arthrose |
| Q:64J,m, | aktivierte Arthrose |
| R:61J,w, | Innenbandzerrung (noch nicht abgeschlossen) |
| S:50J,w | Distorsion nach Sturz |

**Tabelle 4**

| Patient | Anzahl Behanglungen | Behandlungszeit (Tage) | Erfolg | | | | |
|---|---|---|---|---|---|---|---|
| | | | Ruheschmerz | | Belastungsschmerz | | |
| | | | Vor | nach | vor | nach, %VAS | |
| A | 3 | 28 | 50 | 50 | 60 | 60 | - |
| B | 4 | 42 | 55 | 30 | 65 | 35 | + |
| C | 2 | 3 | 50 | 0 | 70 | 20 | + |
| D | 1 | 1 | 50 | 0 | 70 | 0 | + |
| E | 1 | 1 | 45 | 0 | 60 | 5 | + |
| F | 5 | 25 | 45 | 0 | 60 | 0 | + |
| G | 11 | 75 | 70 | 0 | 70 | 0 | + |
| H | 10 | 21 | 85 | 15 | 85 | 30 | + |
| l | 30 | 150 | 75 | 0 | 85 | 15 | + |
| K | 9 | 44 | 70 | 60 | 80 | 70 | - |
| K | 12 | 44 | 50 | 0 | 75 | 5 | + |
| L | 2 | 7 | 0 | 0 | 50 | 15 | + |
| L | 12 | 41 | 30 | 0 | 60 | 15 | + |
| M | 5 | 21 | 35 | 0 | 55 | 5 | + |
| N | 12 | 27 | 50 | 0 | 65 | 0 | + |
| O | 3 | 8 | 70 | 70 | 60 | 30 | +/- |
| P | 5 | 15 | 40 | 10 | 65 | 20 | + |
| Q | 5 | 24 | 30 | 0 | 60 | 0 | + |
| R | 6 | 15 | 50 | 30 | 70 | 50 | + |
| S | 4 | 5 | 30 | 0 | 60 | 15 | + |

## Patentansprüche

1. Zubereitung in Form einer Mikro- Emulsion, enthaltend ein Vehikel -System aus einem oder mehreren Mitteln mit an sich hautreizender, insbesondere hyperämiesierender Wirkung, und einem oder mehreren Mitteln mit lokalanästhetischer Wirkung, wobei das die Haut reizende(n) Mittel ausgewählt ist aus Capsaicinoiden (Vanillyl-Amide (ungesättigter) aliphatischer C₈-C₁₃-Carbonsäuren).

2. Zubereitung gemäß Anspruch 1, **dadurch gekennzeichnet, dass** das die Haut reizende(n) Mittel ausgewählt ist aus Capsaicin.

3. Zubereitung gemäß Anspruch 1 oder 2, **dadurch gekennzeichnet, dass** sie in Form einer Tensidhaltigen Mikro-Emulsion mit einer wässrigen und einer Lipoidphase vorliegt, einen oder mehrere indikationsbezogene Wirksubstanz(en) und ein Vehikel- System mit einem oder mehreren Mitteln mit an sich hautreizender Wirkung und einem oder mehreren Mitteln mit lokalanästhetischer Wirkung aufweist.

4. Zubereitung gemäß einem der Ansprüche 1 bis 3, **dadurch gekennzeichnet, dass** das oder die Lokalanästhetika ausgewählt ist / sind aus Lidocain, Procain, Bupivacain, Polidocanol, Benzocain, Tetracain, Prilocain, Mepivacain, Etidocain, Levobupivacain, Ropivacain, Articain, Fomocain oder Mischungen hiervon.

5. Zubereitung nach einem der Ansprüche 1 bis 4, **dadurch gekennzeichnet, dass** sie weiterhin Cotenside und / oder Zusatzstoffe, ausgewählt aus Vesikelbildnern; Diffusionsverstärkern, Feuchthaltemitteln, Konservierungsmitteln oder Mischungen hiervon enthält.

6. Zubereitung nach Anspruch 5, **dadurch gekennzeichnet, dass** sie 12 bis 30 Gew. %einer Ö-(Lipid-)phase,1 bis 35 % nichtionischer Tenside; 0,01 bis 15 Gew.% Transport- Vehikel; 1 bis 37 Gew.% Zusatzstoffe, ausgewählt aus Cotensiden, Vesikelbildnern, Diffusionsverstärkern, 1 bis 20 Gew.% Wasser oder wässrige Phase sowie 0,01 bis 25 Gew.% eines oder mehrerer indikatonsbezogener Wirkstoffe aufweist.

7. Zubereitung gemäß einem der Ansprüche 5 oder 6, **dadurch gekennzeichnet, dass** sie als Diffusionsverstärker ein oder mehrere Substanzen, ausgewählt aus Terpenoiden wie Limonen, Menthol, Azonen, Ölsäure, Isopropyl-Myristat, Propylenglycol, Harnstoff, Dimethyl-Acetamid, Dimethyl-Formamid, alkanisiertes Pyrrolidon, Cholesterol und Dimethyl-Sulfoxid(DMSO), aufweist.

8. Zubereitung gemäß einem der Ansprüche 1 bis 7, **dadurch gekennzeichnet, dass** als Cotensid ein aliphatischer Alkohol, ausgewählt aus Ethanol, Propanol, Isopropanol, Butanol, Hexanol oder Mischungen hiervon enthalten ist.

9. Zubereitung gemäß einem der Ansprüche 1 bis 8, **dadurch gekennzeichnet, dass** als Tenside(e) ein oder mehrere Substanzen, ausgewählt aus nichtionischen Tensiden mit einem HLB- Wert von 2-8 in Kombination mit einem oder mehreren Tensiden mit einem HLB- Wert von 9-18 enthalten sind.

10. Zubereitung gemäß einem der Ansprüche 6 bis 9, **dadurch gekennzeichnet, dass** sie mit Wasser oder einer wässrigen Phase gemischt ist, und je nach Wassermenge flüssig (ca. 1-20 Gew. %) oder gelförmig bis fest (> 20 bis 50 Gew.%) ist.

11. Zubereitung gemäß einem der Ansprüche 1 bis 10, **dadurch gekennzeichnet, dass** der indikationsbezogene Wirkstoff ausgewählt ist aus entzündungshemmenden Mitteln, antiviralen Mitteln, anti-Akne- Mitteln, Schmerzmitteln, Anti-Thrombose- Mitteln, anti- Gicht-Mitteln, anti-Arthritis-Mitteln, anti-Herpes zoster-Mitteln, anti-Neuralgie-Mitteln, anti-Neuropathie-Mitteln, anti-Thrombophlebitis-Mitteln, juckreizstillenden Mittein-, anti-allergischen, anti-infektiösen, anti-hämorrhagischen, anti-histaminischen, anti-koagulierenden, anti-mykotischen, aquaretischen, ödemprotektiven, anti-oxidativen, antiparasitischen, anti-rheumatischen, anti-septischen, anti-infektiösen, spasmolytischen, keratolytischen, Mitose-hemmenden, Muskel-relaxierenden und vasodilatorischen Mitteln oder Mischungen hiervon.

12. Zubereitung gemäß Anspruch 11, **dadurch gekennzeichnet, dass** der indikationsbezogene Wirkstoff ausgewählt ist aus Colchicin, Aciclovir, Teebaum-Öl, Podophyllotoxin, Melissen-Öl, Glycyrrhetinsäure, Dictophenac, Cardiospermum-Urtinktur, Tretinoin, Kamille blau, Thymian-Öl, Zimt-Öl, Birken-Öl, Digitoxin, Heparin, Extraktum hippocastani (Aescin), ätherische Öle von Zypresse, Wacholder, Rosmarin, Niouliöl, Patchouliöl, Tea Treeöl, Geranienöl, Basilikumöl, Ysopöl, Fenchelöl, Manukaöl, Flavonoide, Salicylate, Harnstoff, Retinoide, Dithronal(Cignolin), Mahonia-Urtinktur, Teersubstanzen (Kresole, Phenole), Beinwellextrakt; Vitamine oder geeigneten Mischungen hiervon.

13. Zubereitung gemäß Anspruch 11. **dadurch gekennzeichnet, dass** auch ein oder mehrere indikationsbezogene Wirkstoffe, ausgewählt aus Salicylamid, Acetylsalicylsäure, Satsalat, Iridometacin, Diclofenac, Fenbufen, Ibuprofen, Ketoprofen, Piroxicam, Harnstoff, Thymol, Chlorhexidin, Erythromycin, Clindamycin, Tetracycline, Glucocorticoide, Hirudin und Derivate, Heparine, Clotrimazol, Isoconazol, Ciclopirox, Roter Weinlaub-Extrakt, Glutathion, Cystein, Coenzym Q10, alpha-Lipon-säure, Carvacrol, Campher, Eugenol, Zimt-Aldehyd, Botulinum-Toxinen, Nitroglycerin eingesetzt sind.

14. Zubereitung gemäß einem der Ansprüche 1 bis 13, **dadurch gekennzeichnet, dass** sie sich in flüssiger Form in einem Spender mit einem geeigneten Pumpmechanismus befindet.

15. Zubereitung gemäß einem der Ansprüche 1 bis 13, **dadurch gekennzeichnet, dass** sie sich zusammen mit einem geeigneten Treibgas, insbesondere Luft oder Treibgas, oder mit Sauerstoff angereichertem Treibgas in einem Aerosolbehältnis befindet.

16. Zubereitung gemäß einem der Ansprüche 1 bis 15, **dadurch gekennzeichnet, dass** sie extern topisch applizierbar ist.

17. Verwendung einer Zubereitung gemäß einem der Ansprüche 1 bis 16 zur Herstellung eines Mittels zur externen dermatologischen oder medizinischen Anwendung bei Menschen oder Tieren zur topischen Behandlung einer allergisch, bakteriell, immunologisch, durch äußere Einflüsse beeinträchtigten, irritierten oder beschädigten und degenerierten Haut, oder von entzündlichen Prozessen in unterliegenden geweblichen Bereichen von Säugern.

18. verwendung gemäß Anspruch 17 zur externen Anwendung bei entzündlichen Störungen wie Gicht, Arthritis, Akne, Psoriasis, venösen Erkrankungen, Herpes Zoster, Neurodermitis, Schmerzen, Koagulationsstörungen, Tendinopathien, Neuropathien, aktivierten Arthrosen, Allergien, Muskelverspannungen, viralen, bakeriellen, mykotischen Infektionen, komplexes regionales Schmerz- Syndrom (CRPS), Thrombosen.

19. Verwendung gemäß Anspruch 17, **dadurch gekennzeichnet, dass** die entzündliche Störung Thrombo- Phlebitis ist und als indikationsbezogener Wirkstoff Digitoxin, Heparin, Extractum hippocastan (Aescin) oder Zypressen-, Wacholder-, Rosmarin- Öl, Flavonoide ausgewählt ist.

20. Verwendung gemäß einem der Ansprüche 17, 18 oder 19, **dadurch gekennzeichnet, dass** die Zubereitung tropfenweise, durch Sprühen und / oder durch Einreiben auf das anzuwendende Areal aufgetragen wird.

## Claims

1. A preparation in the form of a microemulsion containing a vehicle system prepared from one or more agents having an intrinsic skin irritant action, in particular circulation-boosting action, and one or more agents having a local anaesthetic action, wherein the skin irritant agent is selected from capsaicinoids (vanillyl amides of (unsaturated) aliphatic C₈-C₁₃ carboxylic acids).

2. A preparation according to claim 1, **characterised in that** the skin irritant agent is selected from capsaicin.

3. A preparation according to claim 1 or claim 2, **characterised in that** it assumes the form of a surfactant-containing microemulsion with an aqueous phase and a lipoid phase, one or more active substance(s) appropriate for the indication and a vehicle system with one or more agents having an intrinsic skin irritant action and one or more agents having a local anaesthetic action.

4. A preparation according to any one of claims 1 to 3, **characterised in that** the local anaesthetic(s) is/are selected from lidocaine, procaine, bupivacaine, polidocanol, benzocaine, tetracaine, prilocaine, mepivacaine, etidocaine, levobupivacaine, ropivacaine, articaine, fomocaine or mixtures thereof.

5. A preparation according to any one of claims 1 to 4, **characterised in that** it furthermore contains cosurfactants and/or additives selected from vesicle formers; diffusion promoters, humectants, preservatives, or mixtures thereof.

6. A preparation according to claim 5, **characterised in that** it comprises 12 to 30 wt.% of an oil (lipid) phase, 1 to 35% nonionic surfactants; 0.01 to 15 wt.% transport vehicle; 1 to 37 wt.% additives selected from cosurfactants, vesicle formers, diffusion promoters, 1 to 20 wt.% water or aqueous phase and 0.01 to 25 wt.% of one or more active ingredients appropriate for the indication.

7. A preparation according to any one of claims 5 or 6, **characterised in that**, as diffusion promoter, it comprises one or more substances selected from terpenoids such as limonene, menthol, azonine, oleic acid, isopropyl myristate, propylene glycol, urea, dimethylacetamide, dimethylformamide, alkanised pyrrolidone, cholesterol and dimethyl sulfoxide (DMSO).

8. A preparation according to any one of claims 1 to 7, **characterised in that**, as cosurfactant, it contains an aliphatic alcohol selected from ethanol, propanol, isopropanol, butanol, hexanol or mixtures thereof.

9. A preparation according to any one of claims 1 to 8, **characterised in that**, as surfactants(s), it contains one or more substances selected from nonionic surfactants with an HLB value of 2-8 in combination with one or more surfactants with an HLB value of 9-18.

10. A preparation according to any one of claims 6 to 9, **characterised in that** it is mixed with water or an aqueous phase and, depending on the quantity of water, is in liquid (approx. 1-20 wt.%) or gel to solid form (> 20 to 50 wt.%).

11. A preparation according to any one of claims 1 to 10, **characterised in that** the active ingredient appropriate for the indication is selected from antiinflammatory agents, antiviral agents, antiacne agents, analgesics, antithrombotic agents, antigout agents, antiarthritis agents, anti-Herpes zoster agents, antineuralgia agents, antineuropathy agents, antithrombophlebitis agents, itch-relieving agents, antiallergic, antiinfective, antihaemorrhagic, antihistaminic, anticoagulant, antimycotic, aquaretic, oedema-protective, antioxidant, antiparasitic, antirheumatic, antiseptic, antiinfective, spasmolytic, keratolytic, mitosis-inhibiting, muscle-relaxant and vasodilatory agents or mixtures thereof.

12. A preparation according to claim 11, **characterised in that** the active ingredient is selected from colchicine, aciclovir, tea tree oil, podophyllotoxin, lemon balm oil, glycyrrhetic acid, diclofenac, cardiospermum mother tincture, tretinoin, chamomile blue, thyme oil, cinnamon oil, birch oil, digitoxin, heparin, horse chestnut extract (aescin), essential oils of cypress, juniper, rosemary, niaouli oil, patchouli oil, tea tree oil, geranium oil, basil oil, hyssop oil, fennel oil, manuka oil, flavonoids, salicylates, urea, retinoids, dithronal (cignolin), mahonia mother tincture, tar substances (cresol, phenols), comfrey extract; vitamins or suitable mixtures thereof.

13. A preparation according to claim 11, **characterised in that** one or more active ingredients appropriate for the indication are also used which are selected from salicylamide, acetylsalicylic acid, salsalate, iridomethacin, diclofenac, fenbufen, ibuprofen, ketoprofen, piroxicam, urea, thymol, chlorhexidine, erythromycin, clindamycin, tetracyclines, glucocorticoids, hirudin and derivatives, heparins, clotrimazole, isoconazole, ciclopirox, red grape leaf extract, glutathione, cysteine, coenzyme Q10, alpha-lipoic acid, carvacrol, camphor, eugenol, cinnamaldehyde, botulinum toxins, nitroglycerine.

14. A preparation according to any one of claims 1 to 13, **characterised in that** it is present in liquid form in a dispenser with a suitable pump mechanism.

15. A preparation according to any one of claims 1 to 13, **characterised in that** it is present in an aerosol container together with a suitable propellant gas, in particular air or propellant gas, or oxygen-enriched propellant gas.

16. A preparation according to any one of claims 1 to 15, **characterised in that** it may be applied by external topical administration.

17. Use of a preparation according to any one of claims 1 to 16 for producing an agent for external dermatological or medical application on humans or animals for topical treatment of skin impaired, irritated or damaged and degenerated by allergic, bacterial or immunological causes or by external influences, or by inflammatory processes in underlying tissue regions in mammals.

18. Use according to claim 17 for external application in inflammatory disorders such as gout, arthritis, acne, psoriasis, venous diseases, *Herpes zoster,* neurodermatitis, pain, coagulation disorders, tendinopathies, neuropathies, activated arthroses, allergies, muscle tension, viral, bacterial, mycotic infections, complex regional pain syndrome (CRPS), thromboses.

19. Use according to claim 17, **characterised in that** the inflammatory disorder is thrombophlebitis and the active ingredient appropriate for the indication which is selected is digitoxin, heparin, horse chestnut extract (aescin) or cypress, juniper, rosemary oil or flavonoids.

20. Use according to any one of claims 17, 18 or 19, **characterised in that** the preparation is administered dropwise, by spraying and/or by rubbing into the area for application.

## Revendications

1. Composition sous forme d'une microémulsion, contenant un système de véhicule constitué d'un ou de plusieurs agents présentant un effet en soi irritant pour la peau, en particulier hyperémiant, et d'un ou de plusieurs agents dotés d'un effet anesthésiant local, le ou les agents irritant la peau étant choisis parmi les capsaicinoïdes (vanillylamides d'acides carboxyliques aliphatiques (insaturés) en C₈-C₁₃).

2. Composition selon la revendication 1, **caractérisée en ce que** le ou les agents irritant la peau sont choisis parmi la capsaïcine.

3. Composition selon la revendication 1 ou 2, **caractérisée en ce qu'**elle se trouve sous forme d'une microémulsion contenant des agents tensioactifs, présentant une phase aqueuse et une phase lipoïde et une ou plusieurs substances actives relatives à une indication et un système de véhicule contenant un ou plusieurs agents présentant un effet en soi irritant pour la peau et un ou plusieurs agents dotés d'un effet anesthésiant local.

4. Composition selon l'une quelconque des revendications 1 à 3, **caractérisée en ce que** le ou les anesthésiques locaux sont choisis parmi la lidocaïne, la procaïne, la bupivacaïne, le polidocanol, la benzocaïne, la tétracaïne, la prilocaïne, la mépivacaïne, l'étidocaïne, la lévobupivacaïne, la ropivacaïne, l'articaïne, la fomocaïne ou leurs mélanges.

5. Composition selon l'une quelconque des revendications 1 à 4, **caractérisée en ce qu'**elle contient en outre des co-agents tensioactifs et/ou des additifs, choisis parmi les agents de formation de vésicules, les agents de renforcement de la diffusion, les agents de rétention de l'humidité, les conservateurs ou leurs mélanges.

6. Composition selon la revendication 5, **caractérisée en ce qu'**elle présente 12 à 30% en poids d'une phase huileuse (lipidique), 1 à 35% d'agents tensioactifs non ioniques, 0,01 à 15% en poids de véhicule de transport ; 1 à 37% en poids d'additifs, choisis parmi les co-agents tensioactifs, les agents de formation de vésicules, les agents de renforcement de la diffusion, 1 à 20% en poids d'eau ou d'une phase aqueuse ainsi que 0,01 à 25% en poids d'un ou de plusieurs substances actives relatives à une indication.

7. Composition selon l'une quelconque des revendications 5 ou 6, **caractérisée en ce qu'**elle présente, comme agent de renforcement de la diffusion, une ou plusieurs substances choisies parmi les terpénoïdes tels que le limonène, le menthol, l'azonène, l'acide oléique, le myristate d'isopropyle, le propylèneglycol, l'urée, le diméthylacétamide, le diméthylformamide, la pyrrolidone alcanisée, le cholestérol et le diméthylsulfoxyde (DMSO).

8. Composition selon l'une quelconque des revendications 1 à 7, **caractérisée en ce qu'**elle contient comme co-agent tensioactif un alcool aliphatique choisi parmi l'éthanol, le propanol, l'isopropanol, le butanol, l'hexanol ou leurs mélanges.

9. Composition selon l'une quelconque des revendications 1 à 8, **caractérisée en ce qu'**elle contient comme agent(s) tensioactif(s) une ou plusieurs substances choisies parmi les agents tensioactifs non ioniques présentant une valeur BHL de 2-8 en combinaison avec un ou plusieurs agents tensioactifs présentant une valeur BHL de 9-18.

10. Composition selon l'une quelconque des revendications 6 à 9, **caractérisée en ce qu'**elle est mélangée à l'eau ou à une phase aqueuse et qu'elle est, en fonction de la quantité d'eau, liquide (environ 1-20% en poids) ou sous forme de gel à solide (> 20 à 50% en poids).

11. Composition selon l'une quelconque des revendications 1 à 10, **caractérisée en ce que** la substance active relative à une indication est choisie parmi les agents anti-inflammatoires, les agents antiviraux, les agents anti-acné, les antidouleurs, les agents antithrombotiques, les agents anti-goutte, les agents anti-arthrite, les agents anti-herpes zoster, les agents antinévralgiques, les agents anti-neuropathiques, les agents anti-thrombophlébitiques, les agents antiprurigineux, les agents antiallergiques, anti-infectieux, antihémorragiques, antihistaminiques, anticoagulants, antimycosiques, aquarétiques, de protection contre les oedèmes, antioxydants, antiparasitiques, antirhumatismaux, antiseptiques, antiinfectieux, spasmolytiques, kératolytiques, inhibiteurs des mitoses, relaxants musculaires et vasodilatateurs ou leurs mélanges.

12. Composition selon la revendication 11, **caractérisée en ce que** la substance active relative à une indication est choisie parmi la colchicine, l'aciclovir, l'huile de l'arbre à thé, la podophyllotoxine, l'huile de mélisse, l'acide glycyrrhétinique, le diclophenac, la teinture mère de cardiospermum, la trétinoïne, le bleu de camomille, l'huile de thym, l'huile de cannelle, l'huile de bouleau, la digitoxine, l'héparine, l'Extraktum hippocastani (Aescine), les huiles essentielles de cyprès, de genévrier, de romarin, l'huile de niaouli, de patchouli, de l'arbre à thé, de géranium, de basilic, d'hysope, de fenouil, de manuka, les flavonoïdes, les salicylates, l'urée, les rétinoïdes, le dithronal (Cignoline), la teinture mère de mahonia, les substances goudronnées (crésols, phénols), l'extrait de consoude ; les vitamines ou des mélanges appropriés de ceux-ci.

13. Compositions selon la revendication 11, **caractérisée en ce qu'**une ou plusieurs substances actives relatives à des indications, choisies parmi le salicylamide, l'acide acétylsalicylique, le salsalate, l'iridométacine, le diclofénac, le fenbufen, l'ibuprofen, le cétoprofen, le piroxicam, l'urée, l'huile de thym, la chlorhexidine, l'érythromycine, la clindamycine, la tétracycline, les glucocorticoïdes, l'hirudine et ses dérivés, l'héparine, le clotrimazol, l'isoconazol, le ciclopirox, l'extrait de vigne rouge, le glutathion, la cystéine, la coenzyme Q10, l'acide alpha-liponique, le carvacrol, le camphre, l'eugénol, l'aldéhyde cinnamique, les toxines de botulinum, la nitroglycérine sont également utilisées.

14. Composition selon l'une quelconque des revendications 1 à 13, **caractérisée en ce qu'**elle se trouve sous forme liquide dans un distributeur équipé d'un mécanisme à pompe approprié.

15. Composition selon l'une quelconque des revendications 1 à 13, **caractérisée en ce qu'**elle se trouve ensemble avec un gaz propulseur approprié, en particulier de l'air ou un gaz propulseur, ou un gaz propulseur enrichi en oxygène dans un récipient à aérosol.

16. Composition selon l'une quelconque des revendications 1 à 15, **caractérisée en ce qu'**elle est applicable par voie topique externe.

17. Utilisation d'une composition selon l'une quelconque des revendications 1 à 16 pour la préparation d'un agent destiné à une utilisation dermatologique ou médicale externe chez les hommes ou les animaux pour le traitement topique d'une peau altérée, irritée ou abîmée et dégénérée par voie allergique, bactérienne, immunologique ou par des influences extérieures ou par des processus inflammatoires dans les zones tissulaires sous-cutanées de mammifères.

18. Utilisation selon la revendication 17 pour une utilisation externe lors de troubles inflammatoires tels que la goutte, l'arthrite, l'acné, le psoriasis, les maladies veineuses, l'Herpes Zoster, la névrodermite, les douleurs, les troubles de coagulation, les tendinopathies, les neuropathies, les arthroses activées, les allergies, les contractures musculaires, les indications virales, bactériennes, mycosiques, le syndrome locorégional douloureux complexe (CRPS), les thromboses.

19. Utilisation selon la revendication 17, **caractérisée en ce que** le trouble inflammatoire est une thrombophlébite et qu'on choisit comme substance active relative à une indication la digitoxine, l'héparine, l'Extractum hippocastani (Aescine) ou l'huile de cyprès, de genévrier, de romarin ou les flavonoïdes.

20. Utilisation selon l'une quelconque des revendications 17, 18 ou 19, **caractérisée en ce que** la composition est appliquée goutte à goutte, par pulvérisation et/ou par frictionnement sur la zone visée.
